# EUROPEAN PATENT SPECIFICATION

(11) **EP 2 696 802 B1**
(45) Date of publication and mention of the grant of the patent: **11.03.2015**
(21) Application number: 12712700.9
(22) Date of filing: 11.04.2012
(51) Int. Cl.: A61F 2/00, A61M 5/42

(54) **INTRAOCULAR INJECTION DEVICE**
INTRAOKULARE INJEKTIONSVORRICHTUNG
DISPOSITIF D'INJECTION INTRAOCULAIRE

(30) Priority: 15.04.2011 EP 11162721
(43) Date of publication of application: 19.02.2014
(73) Proprietor: Sanofi-Aventis Deutschland GmbH, 65929 Frankfurt am Main (DE)
(72) Inventor: SMITH, Christopher, James, Cheshire Holmes Chapel CW4 7QG (GB); HEIGHTON, David, Crescent Wormit FIFE DD6 8PU (GB)
(74) Representative: Keil & Schaafhausen Patent- und Rechtsanwälte
(86) International application number: PCT/EP2012/056588
(87) International publication number: WO 2012/140089

(56) References cited:
- WO-A1-2008/084063
- WO-A1-2010/085338
- WO-A2-00/07530

## Description

The present invention relates to a drug delivery device, especially an apparatus for an intraocular injection.

### Background

In the field of intraocular injections, it is generally important to visualize the injection site prior to, during and after the injection. However, given the small operating space, such visualization is difficult. US 4,743,234 and US 6,001,082 disclose magnifier means for use with hypodermic syringes. However, the known magnifying means do not improve the visibility of the injection site but rather hinder a direct view of the injection site and are cumbersome in use.

Document WO 2008/084063 A1 discloses an apparatus for intraocular injection comprising a plate adapted for being brought into contact with an eye and guiding means for guiding a needle into the interior of the eye. The plate comprises a cut-out having an edge adapted to be positioned along the limbus delimiting the cornea and the sclera of the eye so as to adjust the position of the guiding means with respect to the limbus. Further, the support comprises a hollow body extending over an aperture and comprising an inner guiding channel. The inner guiding channel is of cylindrical shape and is adapted for receiving a syringe in such a way that the syringe can slide into a guiding channel.

Document WO 00/07530 A2 discloses an apparatus for injecting an agent into a tissue, particularly into thin tissues such as the sclera of the eye for effectively imbedding a needle into the tissue at a predetermined penetration approach angle and penetration distance thereby reducing the risk of penetration the full thickness of the tissue. The apparatus includes a support element and a needle guide platform disposed on the support element with an external support surface and a channel extending therethrough and terminating in an aperture at the support surface. A needle disposed in the channel is axially movable along an axis of injection through the channel, wherein the needle is movable from a first rejected position to an extended position corresponding to the penetration distance.

Document WO 2010/085338 A1 refers to a single package infusion set including including a set for placement at an insertion site with means to inspect the region of the skin immediately surrounding the insertion point to ensure that the site is in good condition.

### Summary of the Invention

In an exemplary embodiment, the present invention comprises a device for intraocular injection comprising a drug delivery device, a foot at a distal end of the drug delivery device for positioning the drug delivery device with respect to an injection site, and a magnifying device coupled to one of the drug delivery device and the foot for magnifying a view of the injection site. The drug delivery device comprises a housing compartment for receiving a cartridge or a syringe. The drug delivery device comprises a spring biasing a needle arrangement of the cartridge in a proximal position.

The magnifying device may comprise a prism element, preferably an annular prism element, having its focus on the injection site. The magnifying device is located at a distal end of the drug delivery device or a proximal end of the foot.

The magnifying device may be an integral part of the drug delivery device or permanently attached to the drug delivery device. In another exemplary embodiment, the magnifying device may be releasably attached to the drug delivery device or the foot.

The foot may include a ring for aligning with the cornea.

### Brief Description of the Drawings

In the following, the invention will be described by way of an example and with reference to the schematic drawings in which:
Figures 6 to 12 do not show embodiments of the invention.
Fig. 1 shows a sectional view of a distal part of a device for intraocular injection according to an exemplary embodiment of the present invention;
Fig. 2 shows a sectional view of the distal part of the exemplary embodiment of the device for intraocular injection shown in Fig. 1,
Fig. 3 shows an isometric view of the distal part of the exemplary embodiment of the device for intraocular injection shown in Fig. 1,
Fig. 4 shows a sectional view of a foot similar to the distal portion of the exemplary embodiment of the device for intraocular injection shown in Fig. 1,
Fig. 5 shows an isometric view of the exemplary embodiment of the foot shown in Fig. 4,
Fig. 6 shows a sectional view of the distal part of a device for intraocular injection,
Fig. 7 shows a sectional view of the distal part of the device for intraocular injection shown in Fig. 6,
Fig. 8 shows an isometric view of the distal part of the device for intraocular injection shown in Fig. 6,
Fig. 9 shows an isometric view of a foot similar to the distal part of the device for intraocular injection shown in Fig. 6,
Fig. 10 shows a sectional view of the foot shown in Fig. 9,
Fig. 11 shows a sectional view of the distal part of a device for intraocular injection , and
Fig. 12 shows a sectional view of the distal part of the device for intraocular injection shown in Fig. 11.

### Detailed Description

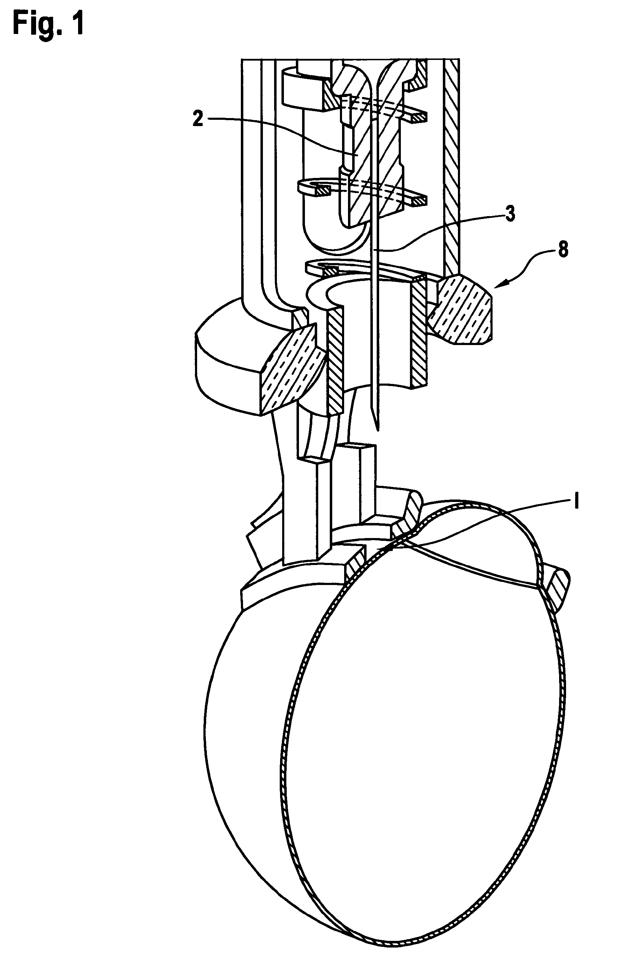

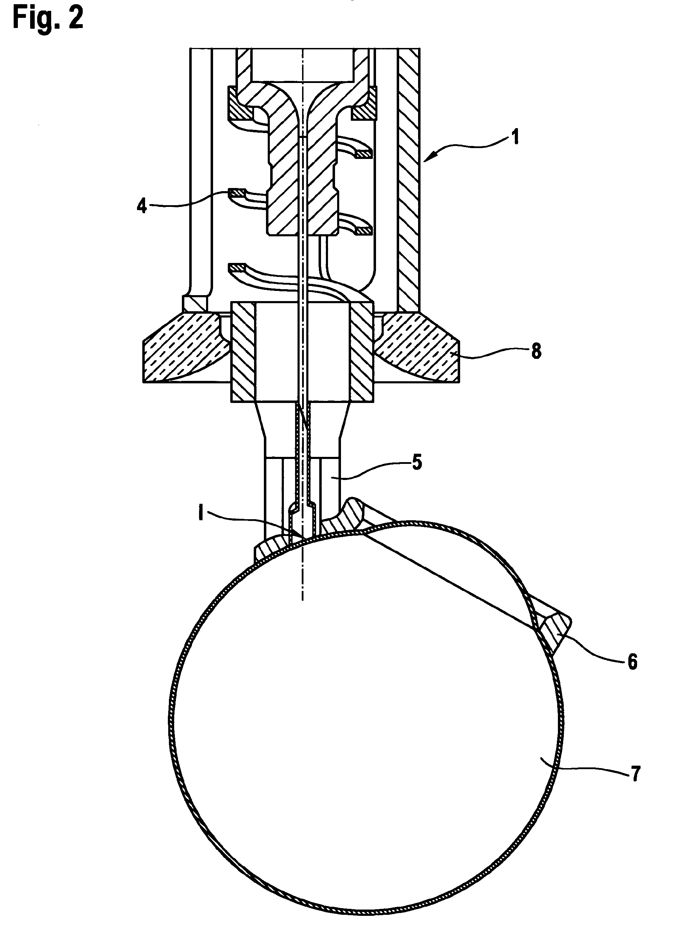

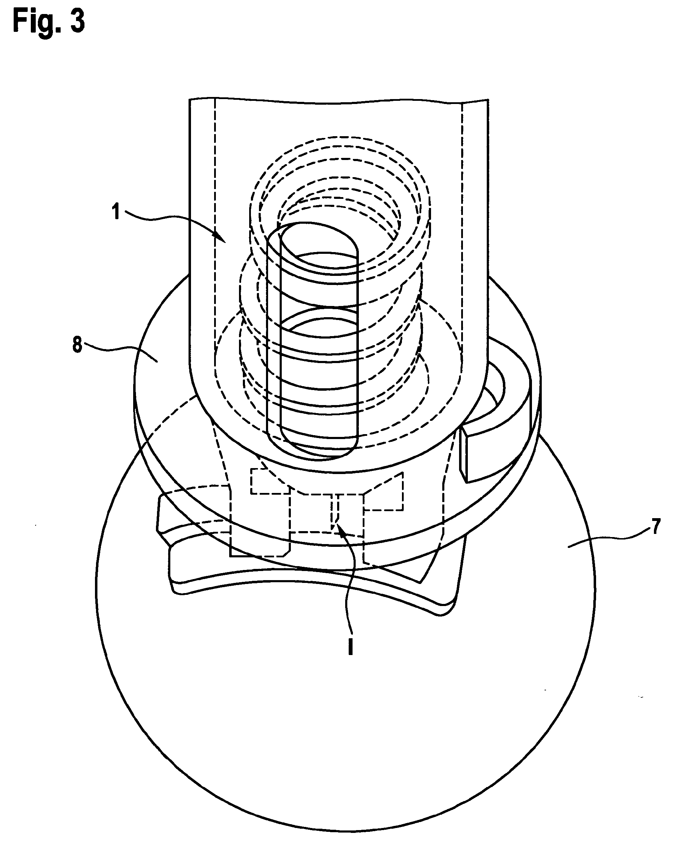

Figures 1 to 3 show an exemplary embodiment of a device for intraocular injection 1, more particularly an intraocular injection device. A body of the device 1 forms a housing compartment for receiving a cartridge 2 (or a drug delivery device, e.g., a syringe) containing a medicinal product, for example steroids or monoclonal antibodies used to treat macular degeneration. A distal end (lower end in the figures) of the cartridge 2 is provided with a needle arrangement 3. Those of skill in the art will understand that the combination of the cartridge 2 and the needle arrangement 3 may be in the form of a prefilled syringe. The drug delivery device may further comprise a spring 4 arranged within the housing compartment to bias the cartridge 2 together with the needle arrangement 3 in the proximal direction. The cartridge 2 together with the needle arrangement 3 may be pushed in the distal direction during injection thus compressing the spring 4 (until it abuts the distal end of the housing compartment) and administering the medicinal product.

In an exemplary embodiment, the device 1 includes a foot 5 or spacer located at its distal end. The foot 5 may be provided with a positioning device to aid the accurate placement of the device relative to an injection site I. In an exemplary embodiment shown in Figures 1 to 3, the foot 5 is provided with a ring 6 which may be sized and shaped to encircle a cornea of a human eye 7. In use, the foot 5 is placed in contact with the eye 7 and the ring 6 is positioned around the cornea. Those of skill in the art will understand that the positioning device may be any size or shape (e.g., circular, semi-circular, arcuate, etc.) which facilitates placement and positioning of the drug delivery device 1. In an exemplary embodiment, the positioning device may have a width which ensures that the injection will not penetrate the cornea, but will be directed through the conjunctiva and sclera into the vitreous body.

In the exemplary embodiment shown in Figures 1 to 3, a magnifying device 8 is provided and positioned to magnify a view of the injection site I. In an exemplary embodiment, the magnifying device 8 may be an annular prism lens formed around a circumference of a distal portion of the drug delivery device 1. In the exemplary embodiment, the use of an annular prism lens may facilitate viewing of the injection site I from multiple angles.

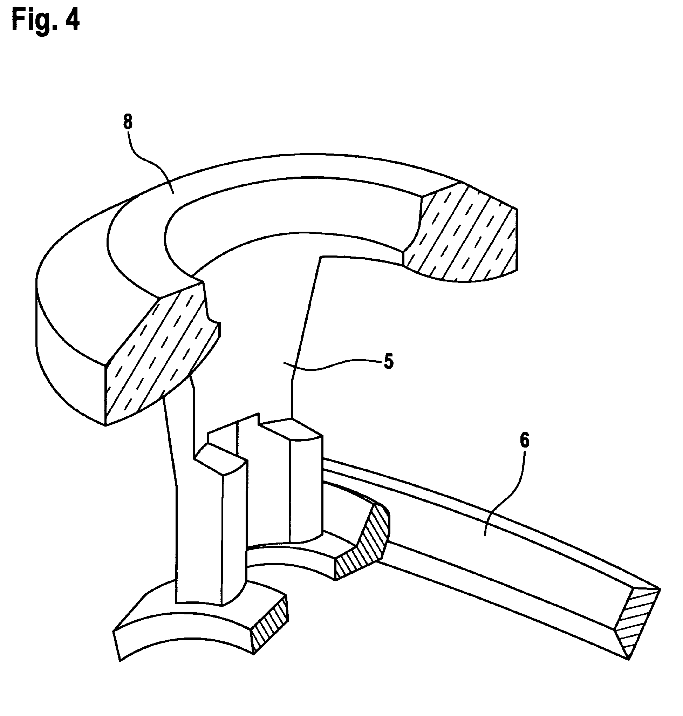

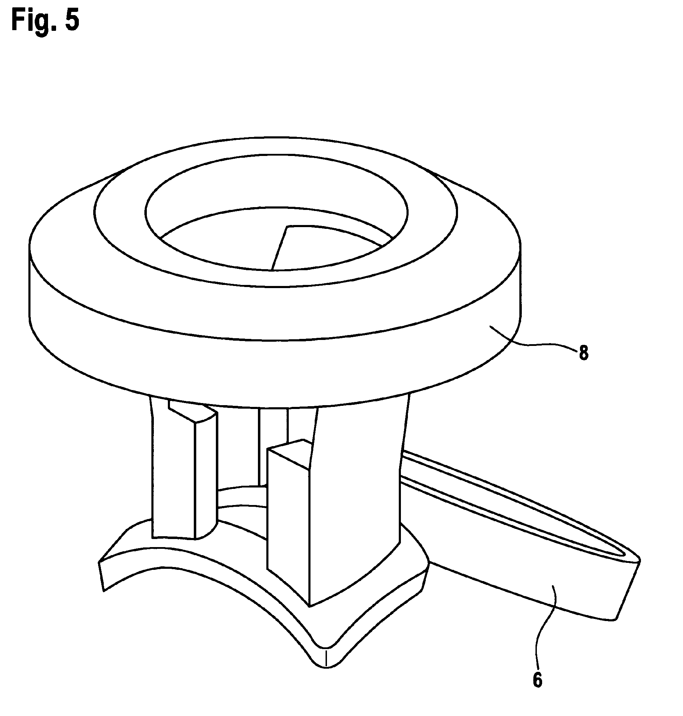

In an exemplary embodiment shown in Figures 4 and 5, the foot 5 is shown as a separate element which may be releasably or permanently attached to, or formed integrally with, the body of a device 1. In an exemplary embodiment in which the foot 5 is separable from the body of the device 1, a proximal portion of the foot 5 may include a coupling mechanism, e.g., threads, snap-fit, bayonet-fit, friction fit, etc., for coupling to a distal end of the body. The coupling mechanism may also include an audible feedback ("click") when it has been secured to the body of the device 1.

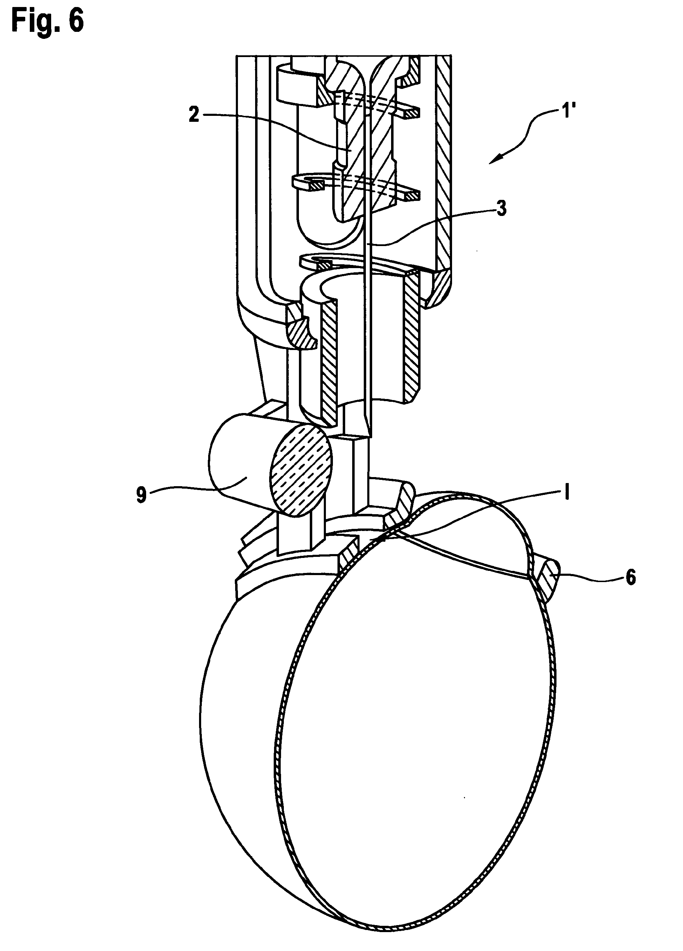

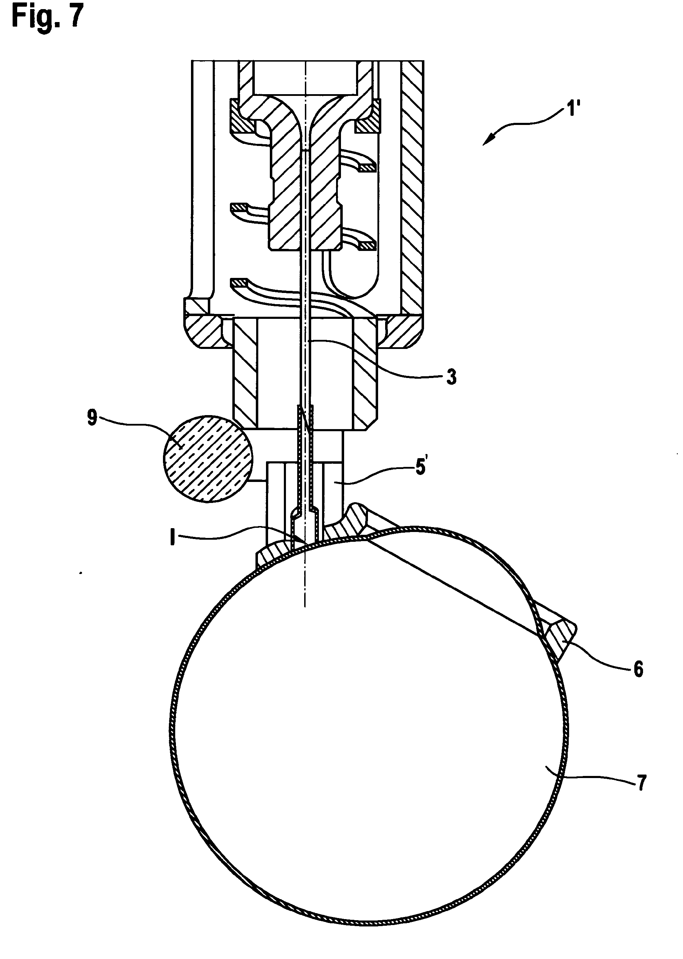

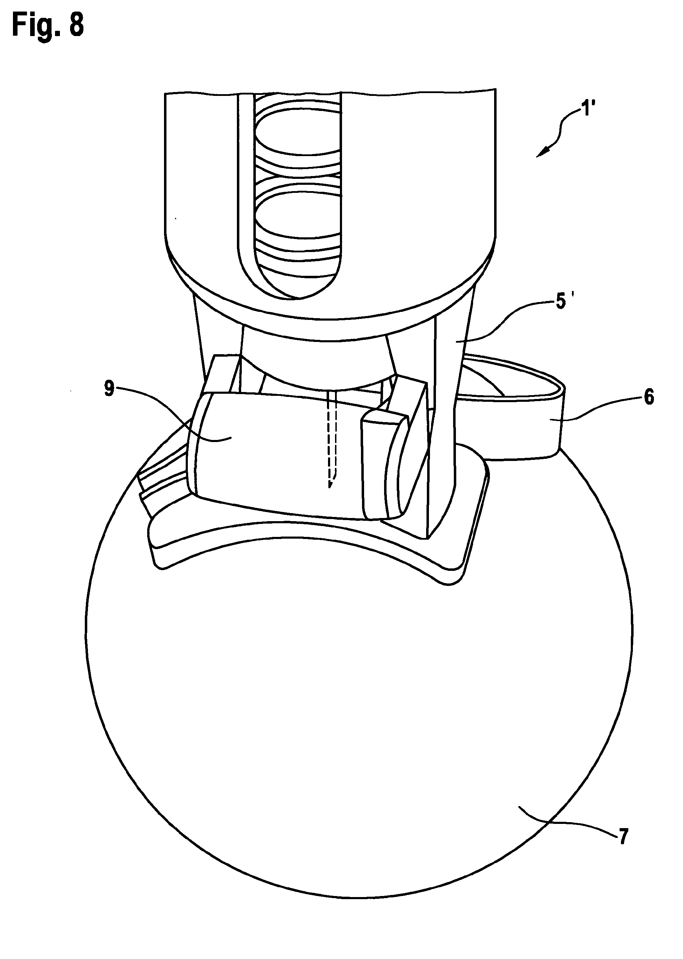

Figures 6 to 8 depict an example not part of the present invention of a device for intraocular injection 1'. The magnifying device may be a barrel lens 9 provided on the foot 5' to provide a magnified view of the injection site I. The barrel lens 9 may be substantially designed as a cylindrical body having an axis extending perpendicular to an axis of the device 1'. The magnifying device may be a disc-shaped lens provided on the foot 5'.

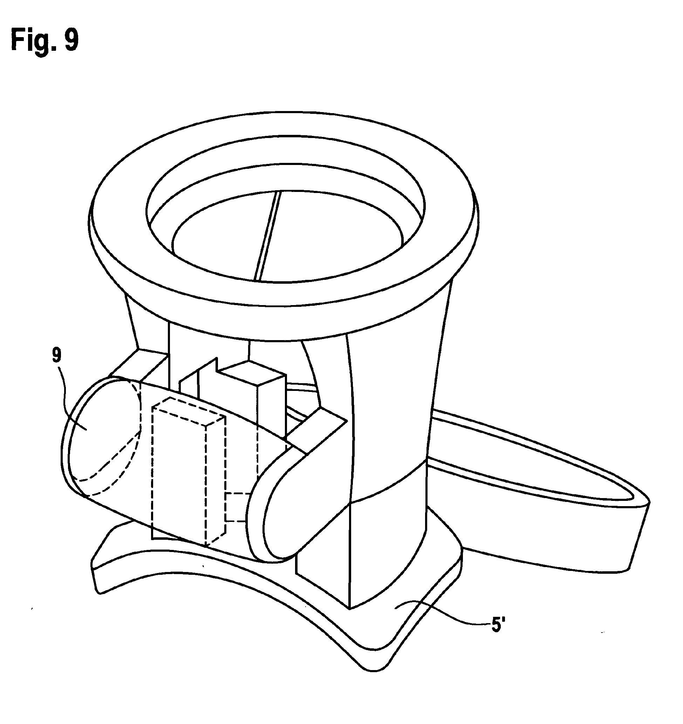

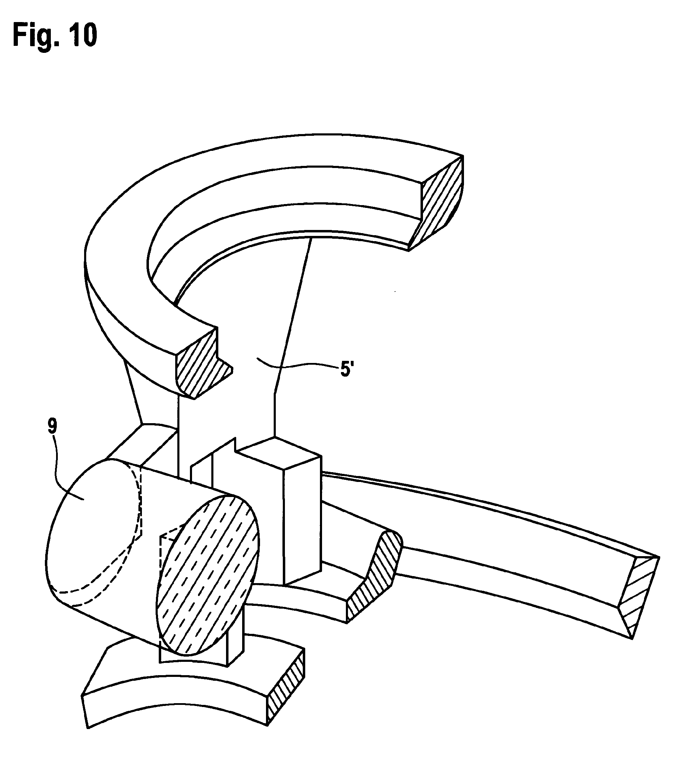

In Figures 9 and 10, the barrel lens 9 may be provided as an integral part of the foot 5'. Alternatively, the barrel lens 9 may be separable from the foot 5'. The foot 5' may either be an integral part of the device 1' or may be provided as a separate part for attachment to the device 1', as described above.

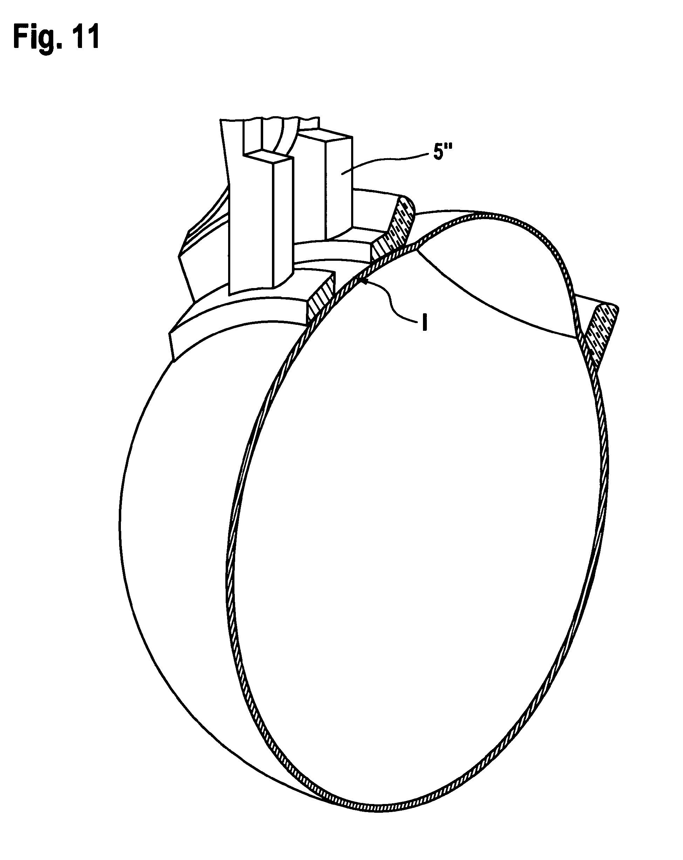

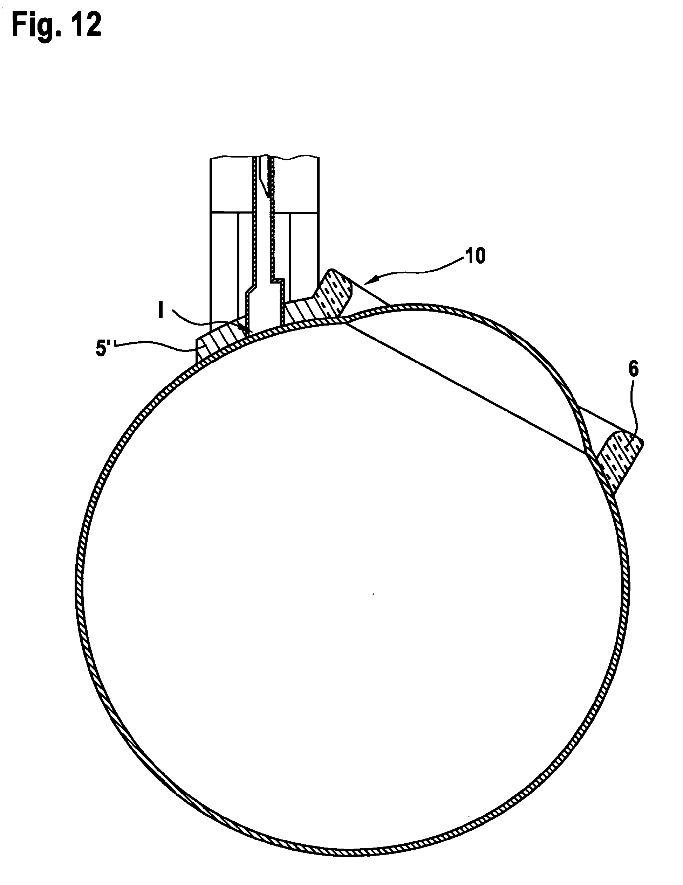

A further example is shown in Figures 11 and 12 depicting a distal portion of a device for intraocular injection including a foot 5" having a magnifying property. For example, a lens element 10 may be integrated into the foot 5", or the foot 5" may be manufactured from a material with a magnifying property.

## Claims

1. A device for intraocular injection comprising:
a drug delivery device (1, 1') comprising a housing compartment for receiving a cartridge (2) or a syringe;
a foot (5) at a distal end of the drug delivery device for positioning the drug delivery device with respect to an injection site;
**characterized in that**
a magnifying device (8) coupled to one of the drug delivery device and the foot (5) for magnifying a view of the injection site is provided, wherein the magnifying device (8) is located at the distal end of the drug delivery device (1, 1') or a proximal end of the foot (5).

2. The device according to claim 1, wherein the drug delivery device comprises a spring (4) biasing a needle arrangement (3) of the cartridge (2) in a proximal position.

3. The device according to any of the preceding claims, wherein the magnifying device (8) comprises a prism element, preferably an annular prism element, having its focus on the injection site.

4. The device according to claims 1 to 2, wherein the magnifying device (8) comprises a barrel lens (9).

5. The device according to claims 2 and 4, wherein the barrel lens (9) is located at a distal end of the drug delivery device or on the foot (5).

6. The device according to claims 1 to 2, wherein the magnifying device (8) comprises a lens (10) located at a distal end of the foot (5).

7. The device according to any of the preceding claims, wherein the magnifying device (8) is an integral part of the drug delivery device or permanently attached to the drug delivery device.

8. The device according to claims 1 to 6, wherein the magnifying device (8) is releasably attached to the drug delivery device or the foot (5).

9. The device according to any of the preceding claims, wherein the foot (5) includes a ring for aligning with the cornea.

## Patentansprüche

1. Vorrichtung zur intraokularen Injektion, umfassend:
eine Medikamenten-Verabreichungsvorrichtung (1, 1'), die eine Gehäusekammer zur Aufnahme einer Patrone (2) oder einer Spritze umfasst,
einen Fuß (5) an einem distalen Ende der Medikamenten-Verabreichungsvorrichtung zur Positionierung der Medikamenten-Verabreichungsvorrichtung bezüglich einer Injektionsstelle,
**dadurch gekennzeichnet, dass**
eine an die Medikamenten-Verabreichungsvorrichtung oder den Fuß (5) gekoppelte Vergrößerungsvorrichtung (8) zur Vergrößerung einer Ansicht der Injektionsstelle vorgesehen ist, wobei die Vergrößerungsvorrichtung (8) am distalen Ende der Medikamenten-Verabreichungsvorrichtung (1, 1') oder an einem proximalen Ende des Fußes (5) angeordnet ist.

2. Vorrichtung nach Anspruch 1, wobei die Medikamenten-Verabreichungsvorrichtung eine Feder (4) umfasst, die eine Nadelanordnung (3) der Patrone (2) in eine proximale Position vorspannt.

3. Vorrichtung nach einem der vorhergehenden Ansprüche, wobei die Vergrößerungsvorrichtung (8) ein Prismenelement, vorzugsweise ein ringförmiges Prismenelement umfasst, dessen Fokus auf der Injektionsstelle ist.

4. Vorrichtung nach Ansprüchen 1 bis 2, wobei die Vergrößerungsvorrichtung (8) eine Tubuslinse (9) umfasst.

5. Vorrichtung nach Ansprüchen 2 und 4, wobei die Tubuslinse (9) an einem distalen Ende der Medikamenten-Verabreichungsvorrichtung oder am Fuß (5) angeordnet ist.

6. Vorrichtung nach Ansprüchen 1 bis 2, wobei die Vergrößerungsvorrichtung (8) eine an einem distalen Ende des Fußes (5) angeordnete Linse (10) umfasst.

7. Vorrichtung nach einem der vorhergehenden Ansprüche, wobei die Vergrößerungsvorrichtung (8) ein integraler Teil der Medikamenten-Verabreichungsvorrichtung oder permanent an der Medikamenten-Verabreichungsvorrichtung angebracht ist.

8. Vorrichtung nach Ansprüchen 1 bis 6, wobei die Vergrößerungsvorrichtung (8) freigebbar an der Medikamenten-Verabreichungsvorrichtung oder dem Fuß (5) angebracht ist.

9. Vorrichtung nach einem der vorhergehenden Ansprüche, wobei der Fuß (5) einen Ring zur Ausrichtung auf die Kornea aufweist.

## Revendications

1. Dispositif d'injection intraoculaire, comprenant:
un dispositif de distribution de médicament (1, 1') comprenant un compartiment de logement pour recevoir une cartouche (2) ou une seringue;
un pied (5) à une extrémité distale du dispositif de distribution de médicament servant à positionner le dispositif de distribution de médicament par rapport à un site d'injection,
**caractérisé en ce que**:
un dispositif de grossissement (8) couplé soit au dispositif de distribution de médicament, soit au pied (5) afin de grossir une vue du site d'injection est prévu, dans lequel le dispositif de grossissement (8) est situé à l'extrémité distale du dispositif de distribution de médicament (1, 1') ou à une extrémité proximale du pied (5).

2. Dispositif selon la revendication 1, dans lequel le dispositif de distribution de médicament comprend un ressort (4) qui pousse un agencement d'aiguille (3) de la cartouche (2) dans une position proximale.

3. Dispositif selon l'une quelconque des revendications précédentes, dans lequel le dispositif de grossissement (8) comprend un élément de prisme, de préférence un élément de prisme annulaire, dont le foyer se trouve sur le site d'injection.

4. Dispositif selon les revendications 1 à 2, dans lequel le dispositif de grossissement (8) comprend une lentille cylindrique (9).

5. Dispositif selon les revendications 2 et 4, dans lequel la lentille cylindrique (9) est située à une extrémité distale du dispositif de distribution de médicament ou sur le pied (5).

6. Dispositif selon les revendications 1 à 2, dans lequel le dispositif de grossissement (8) comprend une lentille (10) qui est située à une extrémité distale du pied (5).

7. Dispositif selon l'une quelconque des revendications précédentes, dans lequel le dispositif de grossissement (8) fait partie intégrante du dispositif de distribution de médicament ou est attaché de façon permanente au dispositif de distribution de médicament.

8. Dispositif selon les revendications 1 à 6, dans lequel le dispositif de grossissement (8) est attaché de façon détachable au dispositif de distribution de médicament ou au pied (5).

9. Dispositif selon l'une quelconque des revendications précédentes, dans lequel le pied (5) comprend un anneau à aligner avec la cornée.
